# EUROPÄISCHE PATENTSCHRIFT

(11) **EP 0 581 207 B1**
(45) Veröffentlichungstag und Bekanntmachung des Hinweises auf die Patenterteilung: **27.08.1997**
(21) Anmeldenummer: 93111809.5
(22) Anmeldetag: 23.07.1993
(51) Int. Cl.: G01N 7/14

(54) **Messeinrichtung zur Erfassung der Gasbeladung oder Dampfdruckes einer Flüssigkeit, insbesondere einer fliessfähigen Kunststoffkomponente**
Apparatus for determining the gas content of a liquid, in particular of a flowable component of a plastic
Appareil pour déterminer la teneur en gaz d'une liquide, en particulier d'un composant fluid d'une matière plastique

(30) Priorität: 25.07.1992 DE 4224616
(43) Veröffentlichungstag der Anmeldung: 02.02.1994
(73) Patentinhaber: SPÜHL AG, CH-9009 St. Gallen (CH)
(72) Erfinder: Strub, Fritz, Dipl.-Ing., CH-9014 St. Gallen (CH)
(74) Vertreter: Riebling, Peter, Dr.-Ing., Patentanwalt

(56) Entgegenhaltungen:
- EP-A- 0 519 273
- DE-A- 3 720 904
- US-A- 4 034 597
- US-A- 4 329 869

## Beschreibung

Gegenstand der Erfindung ist eine Meßeinrichtung zur Erfassung der Gasbeladung oder des Dampfdruckes einer Flüssigkeit, insbesondere einer fließfähigen Kunststoffkomponente, mit den Merkmalen des Oberbegriffs von Patentanspruch 1.

Eine derartige Meßeinrichtung ist z.B in der amerikanischen Patentschrift US-A-4 034 597 gezeigt.

Bei bekannten Meßeinrichtungen zur Erfassung der Gasbeladung oder des Dampfdruckes (siehe z.B. US-A-4 034 597) besteht das Problem, daß man aus einem laufenden Mediumstrom eine Probe des Materials (Flüssigkeit) entnehmen will, um die Gasbeladung oder den Dampfdruck zu messen. Hierbei ist es erforderlich, die entnommene Flüssigkeit hermetisch dicht von dem Mediumstrom abzugrenzen, um die Messung der Gasbeladung oder des Dampfdruckes vornehmen zu können.

Derartige Flüssigkeiten sind z. B. Kunststoffe niedriger oder hoher Viskosität, Kohlenwasserstoffe, wie z. B. Paraffine, Benzin, Petroleum und dgl. mehr.

Die Entnahme der Flüssigkeit aus dem laufenden Volumenstrom bereitet große Schwierigkeiten, denn - wie ausgeführt - muß die entnommene und zur Messung herangezogene Flüssigkeit in genau abgemessenem Volumen aus dem Volumenstrom entnommen werden und muß ferner hermetisch dicht von dem Volumenstrom abgeteilt werden, um die erforderliche Messung der Gasbeladung oder des Dampfdruckes vornehmen zu können.

Bei bekannten Vorrichtungen besteht hierbei der Nachteil, daß man zwar eine Probe aus dem Flüssigkeitsstrom entnehmen kann, daß aber das Zurückführen dieser Meßprobe in den Flüssigkeitsstrom nach Beendigung der Messung Schwierigkeiten bereitet. Man kann nämlich bei den bekannten Anordnungen den Meßraum nicht vollständig entleeren, so daß immer altes Material im Meßraum verbleibt und es schwierig ist, den Meßraum vollständig mit neuem Material zu füllen.

Ebenso bereitet es beim Stand der Technik Schwierigkeiten, den Meßraum überhaupt mit neuem Material zu füllen, und zwar so, daß das alte Material verdrängt wird. Gelingt dies nicht vollständig, dann kommt es zu einer unerwünschten Verfälschung des Meßergebnisses.

Der Erfindung liegt deshalb die Aufgabe zugrunde, eine Meßeinrichtung der eingangs genannten Art so weiterzubilden, daß der Meßraum, welcher ein bestimmtes abgemessenes Volumen aus dem Mediumstrom entnimmt, vor der Messung von dem Volumenstrom gespült wird und ebenso nach der Messung.

Die Lösung der gestellten Aufgabe erfolgt durch das Kennzeichen des Anspruchs 1. Somit ist es wichtig, daß im Bereich eines Meßgehäuses der Durchflußraum vorgesehen ist, der von dem Mediumstrom ständig durchflossen wird. In dem Durchflußraum ist hebund senkbar die Ventilglocke angeordnet, die mit ihrer Unterkante einen Dichtungsschluß mit einem gegenüberliegenden, ebenfalls in den Durchflußraum ragenden Flansch herstellt; ggf. kann auch der Flansch verschiebbar sein.

Durch die Anordnung einer hermetisch abschließbaren Meßkammer im Durchflußraum eines Meßgehäuses wird nun der wesentliche Vorteil erzielt, daß nun bei der Öffnung dieser Meßkammer, die an der einen Seite durch eine heb- und senkbare Ventilglocke begrenzt ist, die Meßkammer nun vollständig von dem Mediumstrom gespült wird.

Damit ist es nun erstmals möglich, direkt aus dem Mediumstrom eine Meßprobe zu entnehmen und in die Meßkammer aufzunehmen, wobei nach erfolgter Messung die Meßkammer geöffnet wird, die Dichtungsglocke demzufolge von dem gegenüberliegenden, gehäusefesten Flansch abgehoben wird und danach die Meßkammer zusammen mit dem gesamten Durchflußraum im Durchfluß des Mediumstroms liegt.

Damit ergibt sich nun der wesentliche Vorteil gegenüber dem Stand der Technik, daß man nicht über Verbindungsleitungen eine Meßprobe aus dem Durchflußraum entnehmen muß, weil die Meßkammer unmittelbar selbst in dem Durchflußraum angeordnet ist. Es entfallen also derartige Verbindungsstutzen oder Blindleitungen, bei denen die Gefahr besteht, daß eine ausreichende Spülung nicht gewährleistet werden kann, so daß im Meßraum noch altes Material einer alten Untersuchungsprobe verbleiben könnte.

Mit der gegebenen technischen Lehre wird also der wesentliche Vorteil erzielt, daß durch die Anordnung der Meßkammer unmittelbar im Durchflußraum eines Meßgehäuses nun erstmals die Meßkammer vollständig gespült werden kann, sofern sie geöffnet ist und die Meßkammer immer eine neue Materialprobe unmittelbar aus dem durchfließenden Mediumstrom entnehmen kann.

Wichtig hierbei ist, daß wenn die Meßkammer in ihrer Meßstellung ist, d. h. wenn sich die Ventilglocke in Dichtungseingriff mit dem gegenüberliegenden, gehäusefesten Flansch befindet, daß dann nach wie vor der Durchflußraum von dem Mediumstrom durchströmt wird, weil dieser Mediumstrom um die Ventilglocke herum verläuft.

Damit ist es nun nicht mehr notwendig, den Eingang und den Ausgang des Meßgehäuses mit entsprechenden Ventilen zu versehen. Derartige Ventile könnten zwar als Dreiweg-Ventile ausgebildet werden, um bei der Veränderung der Ventilstellung ebenfalls einen durchlaufenden Mediumstrom zu erreichen; die Anordnung von Mehrweg-Ventilen bei derartigen Meßgehäusen ist jedoch unerwünscht, weil es mit erhöhtem Aufwand verbunden ist und die Funktionsfähigkeit der Ventile eventuell durch die Art des Materials beeinträchtigt wird, wenn z. B. die Ventile festkleben könnten.

Ebenso könnten die Ventile in unerwünschter Weise die Druckverhältnisse im Meßraum beeinflussen und im übrigen sind sie gegen Zusetzen von Fremdstoffen, Beistoffen oder Füllstoffen im Mediumstrom sehr empfindlich.

Die vorliegende Erfindung hat also den Vorteil, daß man auf irgendwelche Ventile vor oder hinter dem Meßgehäuse verzichten kann und ebenso auf Ventile vor oder hinter der Meßkammer selbst.

Die Meßkammer wird also nach der Erfindung zu einem Teil von der Unterseite der Ventilglocke gebildet, die eine Ausnehmung definiert. Zum anderen wird sie von einem gehäusefesten Flansch definiert, der gegenüberliegend angeordnet ist und der ebenso eine Ausnehmung definiert. Beim Schließen der Ventilglocke auf diesen gehäusefesten Flansch ergibt sich dann die Meßkammer durch die beiden Ausnehmungen, nämlich in der Ventilglocke und in dem gehäusefesten Flansch.

Es wird hierbei bevorzugt, wenn die gesamte Anordnung stehend angeordnet ist, d. h. der Mediumstrom läuft in vertikaler Richtung durch den Durchflußraum und durch die Meßkammer, um Lufteinschlüsse oder Luftblasen im Durchflußraum und im Meßraum zu entfernen.

Damit besteht der Vorteil, daß eventuell im Meßraum sich ansammelnde Blasen nicht die Messung verfälschen können, denn derartige Blasen werden vor dem Schließen der Meßkammer bereits schon ausgetragen und können daher nicht in der Meßkammer eingeschlossen werden und die Messung verfälschen.

Die Wände der Meßkammer sind so geneigt, daß insbesondere im Bereich des Dichtungsschlusses (Unterkante der Ventilglocke im Körperkontakt mit dem gehäusefesten Flansch) geneigte Wände vorhanden sind, so daß sich in diesem Bereich keine Gasblasen festsetzen können.

Nach einer bevorzugten Ausgestaltung der vorliegenden Erfindung findet im Bereich der Meßkammer eine Messung der Gasbeladung des im Mediumstroms enthaltenen Gases statt.

Hierzu ist es vorgesehen, daß eine Seite der Meßkammer entgegen der Kraft einer kalibrierten Feder verschiebbar ausgebildet ist und daß die Verschiebung dieser Wandung ein Maß für die Gasbeladung der Flüssigkeit ist.

Zur Messung der Gasbeladung ist es vorgesehen, daß das in der Meßkammer eingeschlossene Volumen der zu messenden Flüssigkeit fest unter dem Einfluß einer Feder entspannt wird, so daß der Druck der Flüssigkeit in der Meßkammer unter den Zuflußdruck zur Meßkammer gebracht wird. Dieser Druck kann, wenn nötig, auch auf Unterdruck (unterhalb des Atmosphärendruckes) gebracht werden, je nach Auslegung der Feder und des Luftdruckes in Zylinderkammer. Dadurch entgast die Flüssigkeit und die Gaszunahme in der Meßkammer führt zu einer weiteren Verschiebung der einen Wandung der Meßkammer, welche Verschiebung dann durch eine Meßanordnung elektrisch erfaßt wird.

In der vorstehenden Beschreibung wurde angegeben, daß die Ventilglocke heb- und senkbar ausgebildet ist, um einen Dichtungsschluß mit einem gegenüberliegenden, gehäusefesten Flansch zu erreichen, um so die Meßkammer im Bereich des Durchflußraumes zu definieren.

In einer anderen Ausgestaltung der Erfindung ist vorgesehen, daß die Ventilglocke feststehend ausgebildet ist und daß der gegenüberliegend angeordnete Flansch nun nicht mehr gehäusefest ausgebildet ist, sondern daß dieser Flansch verschiebbar oder bewegbar ausgebildet ist, um ebenso in analoger Weise die Meßkammer im Bereich des Durchflußraumes zu bilden und abzuschließen.

Zur Abdichtung der Ventilglocke und einer Wandung der Meßkammer bei entsprechenden Verschiebungen werden sogenannte Rollmembranen verwendet, weil diese Membranen unempfindlich gegen Füll- und Zusatzstoffe des Mediumstromes sind und praktisch keine Reibung erzeugen.

Statt der beschriebenen Rollmembranen können auch Metallbälge verwendet werden, um eine derartige Abdichtungsfunktion zu erreichen.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander. Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von mehrere Ausführungswege darstellende Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Schnitt durch eine Meßeinrichtung nach der Erfindung in der Durchflußstellung;
- Figur 2:: Meßeinrichtung nach Figur 1 in der Meßstellung;
- Figur 3:: ein Öffnungs- und Schließdiagramm der Ventilglocke und der Meßwandung der Meßkammer.

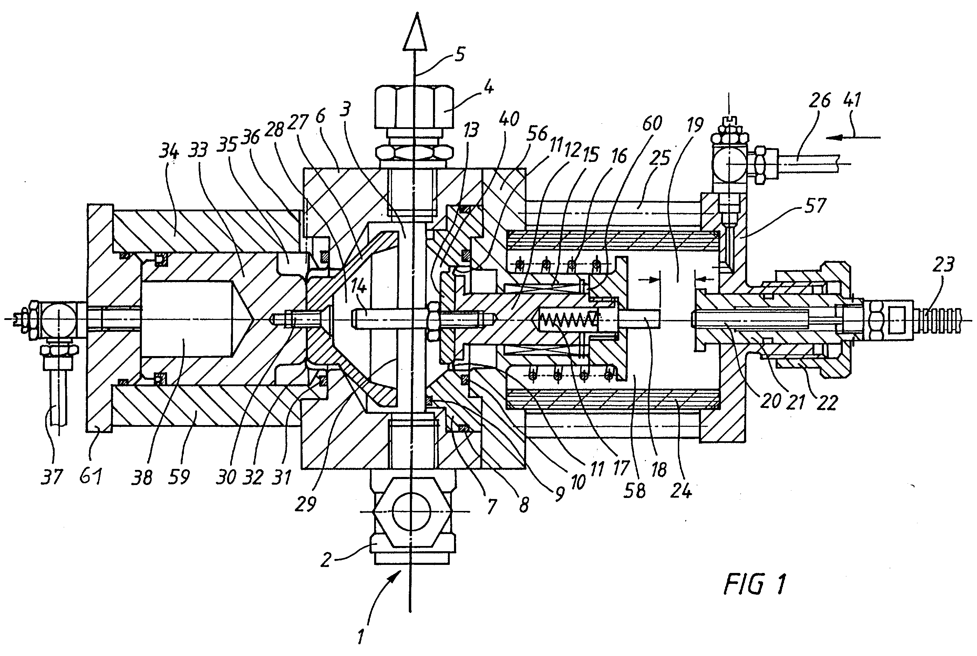

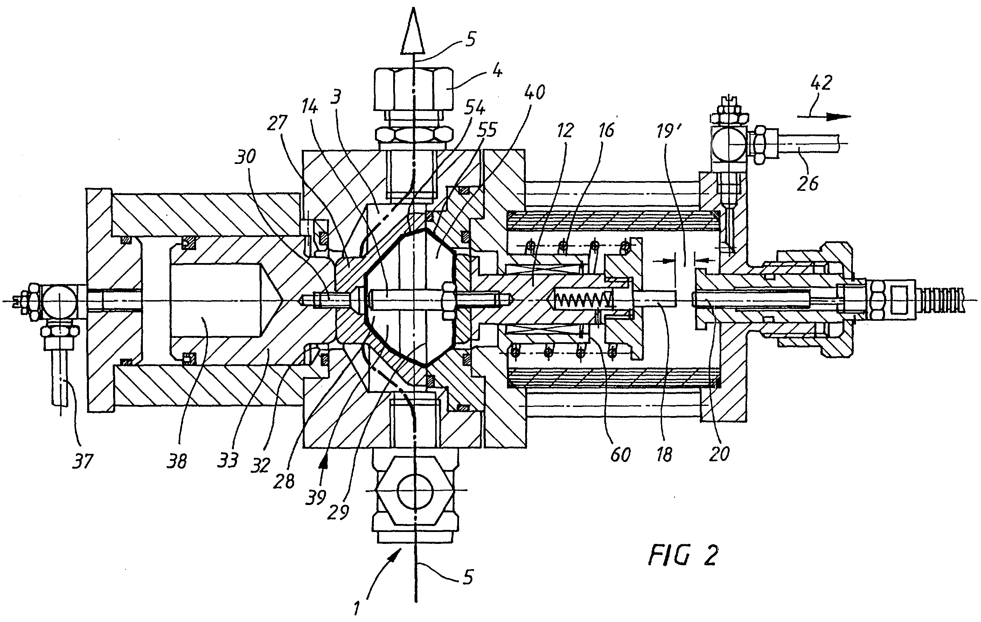

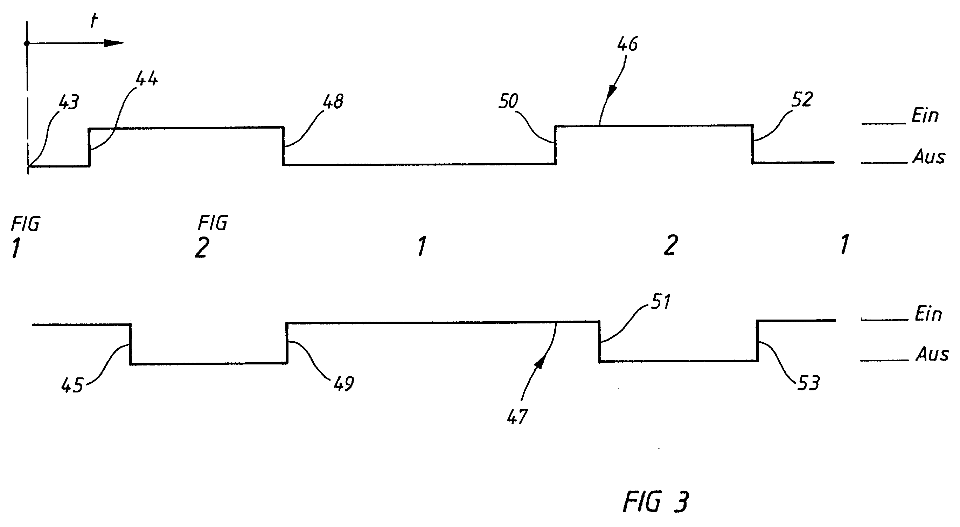

Gemäß Figur 1 sind in einem Meßgehäuse 6 ein Eingang 2 und ein Ausgang 4 einander gegenüberliegend angeordnet, wobei an den Eingang 2 eine Durchflußleitung 1 angeschlossen ist, durch welche ein Mediumstrom 5 über den Eingang 2 zum Ausgang 4 geleitet wird. Dieser Mediumstrom 5 durchströmt einen inneren, zentralen Durchflußraum 3 im Meßgehäuse 6.

Aus diesem Mediumstrom 5 soll nun eine Probe entnommen werden, wobei diese Probe hermetisch dicht von dem Mediumstrom 5 abgeteilt wird, wobei diese Mediumprobe auf einen anderen Druck entspannt werden kann.

Im folgenden Ausführungsbeispiel wird eine Luftbeladungsmeßeinrichtung geschildert, bei der es wesentlich ist, daß eine Wandung der im Durchflußraum 3 gebildeten Meßkammer 39 (Figur 2) verschiebbar ausgebildet ist, um die Luftbeladung der in der Meßkammer 39 angeordneten Meßprobe zu erfassen.

Die eine Seite des Durchflußraumes 3 im Meßgehäuse 6 wird durch einen Flansch 7 gebildet, der stirnseitige Dichtungen 9 aufweist, welche in später zu beschreibenden Weise einen Dichtungsschluß mit der Unterkante 29 einer heb- und senkbar ausgebildeten Ventilglocke 27 erbringen.

Am gehäusefesten Flansch 7 sind noch weitere Dichtungen 8 angeordnet, um den Durchflußraum 3 hermetisch dicht gegenüber der Atmosphäre abzuschließen.

An der Innenseite des Flansches 7 ist hierbei eine Rollmembrane 11 angeordnet, die an der Seite des Flansches 7 in einem Dichtkragen 10 aufgenommen wird.

Die Rollmembrane 11 wird an der radial innenliegenden Seite im Zwischenraum zwischen einem Klemmstück 13 und einem Kolben 12 eingeklemmt und dort dichtend gehalten.

Der Kolben 12 ist die vorher beschriebene verschiebbare eine Wandung der Meßkammer 39.

Der Kolben 12 ist verschiebbar in einem Flansch 56 geführt, der Teil des Meßgehäuses 6 ist, wobei der Flansch 56 einen inneren Ansatz bildet, in dessen Bereich eine Kugelbüchse 15 angeordnet ist, um den Kolben 12 möglichst reibungsarm in Längsrichtung verschiebbar zu führen.

An der Außenseite des Kolbens stützt sich in Zugrichtung eine Feder 16 ab, die sich mit der einen Seite an dem Flansch 56 und mit der anderen Seite an einem Ansatz des Kolbens 12 abstützt.

Damit ist der Kolben 12 durch die Feder 16 im Sinne einer Volumenvergrößerung der Meßkammer 39 vorgespannt.

Im Innenraum des Kolbens 12 ist ein Meßdorn 18 unter der Last einer Feder 17 verschiebbar angeordnet, wobei die vordere Stirnseite des Meßdornes 18 in Verbindung mit der zugeordneten, gegenüberliegenden Stirnseite eines Näherungsschalters 20, den Meßabstand 19 bildet.

Der Näherungsschalter 20 ist hierbei in einer Gewindehalterung im Bereich einer Büchse 21 eingeschraubt, welche Büchse 21 verschiebbar und feststellbar im Bereich eines Flansches 57 durch eine Überwurfmutter 22 gehalten ist. Der Näherungsschalter 20 ist über einen Anschluß 23 mit einer elektrischen Signalverarbeitungsanlage verbunden.

An dem Flansch 57 mündet ein Lufteinlaß 26, über welchen Druckluft in Pfeilrichtung 41 in die Zylinderkammer 58 eingeführt werden kann. Über den Lufteinlaß 26 könnte auch die Zylinderkammer 58 unter Unterdruck gesetzt werden, was die Federwirkung unterstützen würde. Die Feder 16 kann aber nicht gänzlich ersetzt werden, da sonst die verwendeten Rollmembranen umklappen würden. Eine genaue Messung wäre dann unmöglich. Es sei denn, man würde andere Kolbendichtungen verwenden, welche aber nur geringe Reibung aufweisen dürften. Reibung würde die Messung beeinflussen.

Auf der gegenüberliegenden Seite des Kolbens 12 ist ein Abdrückstift 14 angeordnet, der die Öffnungsbewegung der Meßkammer 39 erleichtern soll. Hierbei wird also eine Abstoßbewegung auf die gegenüberliegend angeordnete Ventilglocke 27 ausgeübt.

Die Ventilglocke 27 ist hierbei über eine Schraube 30 mit einem Kolben 33 verbunden, der in der gleichen Weise wie anhand des Kolbens 12 beschrieben wurde, über eine Rollmembrane 32, die in einem umlaufenden Dichtungskragen 31 gefaßt ist, dichtend gegenüber einem Zylinderraum 38 und einem Entlüftungsraum 35 gehalten ist.

Die Dichtungsglocke 27 ist hierbei auf dem Kolben 33 befestigt, welcher Kolben 33 mit Dichtungen 34 gegenüber dem Zylinder 59 abgedichtet sind.

Am Zylinder 59 ist ein Lufteinlaß 37 vorgesehen, über welchen Druckluft in den Zylinderraum 38 eingeleitet werden kann.

Damit sich hinter der Ventilglocke 27 kein Überdruck aufbaut, ist hierbei ein Entlüftungsraum 35 vorgesehen, der über einen Entlüftungsspalt 36 nach außen entlüftet wird.

Der Zylinder ist zwischen einem Deckel 61 und dem Meßgehäuse 6 durch Schrauben eingespannt. Der gegenüberliegende Flansch 57 ist über Bolzen 25 mit dem Flansch 56 verbunden, wobei der Flansch 56 über nicht näher dargestellte Schrauben mit dem Meßgehäuse 6 verbunden ist.

Der Zylinderraum 58 wird hierbei gebildet durch die Wandung 24 eines zylinderförmigen Rohres, welches z. B. aus einem durchsichtigen Kunststoffmaterial ausgebildet sein kann.

Die Meßkammer 39 gemäß Figur 2 wird gebildet einerseits durch eine Ausnehmung 40 in dem rechten, gehäusefesten Flansch 7 und andererseits durch eine innere Ausnehmung 28 im Bereich der etwa C-förmig profilierten Ventilglocke 27.

In der in Figur 1 dargestellten Stellung ist erkennbar, daß der Mediumstrom 5 ungehindert und praktisch turbulenzenfrei durch den Durchflußraum 3 hindurchströmt, weil die Unterkante 29 der Ventilglocke 27 den Volumenstrom nicht behindert.

Sobald aber die Meßkammer 39 geschlossen wird (Figur 2), kommt es ebensowenig zu einer Behinderung des Mediumstromes 5, weil dann - gemäß Figur 2 - der Mediumstrom 5 am Außenumfang der Ventilglocke 27 herum durch den Durchflußraum 3 hindurchströmt.

Der Übergang von der Durchflußstellung in Figur 1 zur Meßstellung nach Figur 2 wird wie folgt bewerkstelligt:

Es wird über den Lufteinlaß 37 Druckluft auf den Zylinderraum 38 gegeben, wodurch der Kolben 33 nach rechts verschoben wird und die Ventilglocke 27 sich mit Ihrer Unterkante 29 abdichtend an der Dichtung 9 des gehäusefesten Flansches 7 an der gegenüberliegenden Seite des Meßgehäuses 6 anlegt.

Es wird also durch die Ventilglocke eine Meßprobe von dem Mediumstrom entnommen und in der jetzt dadurch gebildeten Meßkammer 39 eingeschlossen.

Zur Messung der Luftbeladung der Meßprobe in der Meßkammer 39 ist nun - wie vorher ausgeführt - eine Wandung der Meßkammer 39 verschiebbar ausgebildet und zwar in Form des Kolbens 12.

In der Durchflußstellung gemäß Figur 1 wurde über den Lufteinlaß 26 Luft in Pfeilrichtung 41 in den Zylinderraum 58 eingeführt, wodurch der Kolben 12 mit seinem Ansatz an einem gehäusefesten Anschlag 60 anschlägt.

In der Meßstellung gemäß Figur 2 wird nun über den Lufteinlaß 26 Luft in Pfeilrichtung 42 entspannt, wodurch die Feder 16 den Kolben 12 nach rechts bewegt und zwar von dem gehäusefesten Anschlag 60 entfernt.

Dadurch kommt es zu einer Druckentlastung des in der Meßkammer 39 eingeschlossenen Volumens, wodurch es nun zu einer Gasentladung kommt und Gasblasen in dieser Meßkammer 39 entstehen, welche dafür sorgen, daß der Kolben 12 noch weiter nach rechts unter dem Einfluß der Feder 16 verschoben wird.

Hierdurch nähert sich der Meßdorn 18 dem Näherungsschalter 20, wodurch die Meßstrecke 19 kleiner wird und diese Verringerung der Meßstrecke beim Übergang von Figur 1 auf Figur 2 ist das Maß für die Gasbeladung der in der Meßkammer 39 eingeschlossenen Volumenprobe.

Aus Sicherheitsgründen ist der Meßdorn 18 verschiebbar in dem Innenraum des Kolbens 12 geführt und unter der Kraft der Feder 17 gehalten, um ein unerwünschtes Aufschlagen und damit eine Beschädigung des Näherungsschalters 20 durch den Meßdorn 18 zu vermeiden.

Wichtig ist, daß beim Übergang von der Durchflußstellung in Figur 1 in die Meßstellung nach Figur 2 keine Gasblasen bei sich schließender Meßkammer 39 in der Meßkammer eingefangen werden, weil der Mediumstrom 5 vertikal von unten ansteigend nach oben durch den Durchflußraum 3 strömt und die Wände 54, 55 zwischen der Ventilglocke 27 und dem Flansch 7 angeschrägt sind, so daß dort ansammelnde Gasblasen in den Durchflußraum 3 entlassen werden.

In Figur 3 oben ist das Öffnungs- und Schließdiagramm der Ventilglocke dargestellt, welches der Beaufschlagung des Zylinderraumes 38 mit Druckluft über den Lufteinlaß 37 entspricht. Unten ist das Belastungsdiagramm 47 des Kolbens mit Druckluft über den Luftanschluß 26 dargestellt.

Auf der Abszisse ist die Zeitachse aufgetragen.

Zu einem beliebigen Zeitraum 0 ist bei Position 43 der Kolben 33 geöffnet, d. h. der Zylinderraum 38 ist von Druckluft entlastet und die Ventilglocke 27 wird unter dem Druck des Mediumstromes 5 in ihrer Offenstellung gehalten.

Es wird über den Lufteinlaß 37 Druckluft in den Zylinderraum 38 eingeführt, wodurch der Kolben 33 in seine Schließposition nach rechts verschoben wird und hierbei im Diagramm 46 die Position 44 eingenommen wird.

Um eine gewisse Verzögerungszeit von 1 bis 2 Sekunden wird die vorher in dem Zylinderraum 58 vorhandene Druckluft über den Lufteinlaß 26 in Pfeilrichtung 42 entfernt, wodurch der Kolben bei Position 45 unter Einfluß der Feder 16 in seine Meßausgangsstellung nach Figur 2 gebracht wird. Der Abdrückstift 14 liegt vorerst noch an der Ventilglocke 27 an, hebt jedoch entsprechend der Meßbewegung des Kolbens 12 ab.

Nach Erreichen der Position 45 erfolgt eine Meßdauer von z. B. 30 bis 120 Sekunden, in der die Meßkammer 39 hermetisch dicht abgeschlossen ist.

Bei Position 48 und 49 werden der Kolben 33 durch Entlastung der Druckluft aus dem Lufteinlaß 37 geöffnet und bei Position 49 wird wiederum Druckluft in Pfeilrichtung 41 in den Lufteinlaß 26 gegeben, so daß der Kolben 12 wiederum mit seinem Ansatz auf den gehäusefesten Anschlag 60 auffährt.

Wichtig ist hierbei, daß der Abdrückstift 14 in diesem Zustand die Ventilglocke 27 anhebt und nach links verschiebt, so daß der nun wirkende Mediumstrom 5 die Ventilglocke 27 vollständig in ihrer Öffnungsstellung nach Figur 1 verschieben kann.

Auf der Zeitachse hinter den Positionen 48 und 49 erfolgt ein Durchflußintervall, welches in seiner Länge wählbar ist, z. B. im Bereich von 1 bis 10 Minuten, damit ein genügender Flüssigkeitsaustausch und eine genügende Spülung in der nun geöffneten Meßkammer 39 stattfindet.

Bei den Positionen 50 und 51 wiederholen sich die Vorgänge, die bereits schon anhand der Positionen 54 und 45 beschrieben wurden und die Positionen 52 und 53 entsprechen hierbei dann den vorher beschriebenen Positionen 48 und 49.

Wichtig bei der gesamten Meßeinrichtung ist also, daß im Bereich eines von dem Mediumstrom frei durchflossenen Durchflußraumes 3 eine hermetisch schließbare Meßkammer 39 angeordnet ist, die dann beim Öffnen von dem Mediumstrom 5 frei durchspült wird. Die Bildung von Toträumen, die Anlagerung von Füll- oder Beistoffen im Bereich der Meßkammer wird damit Praktisch vermieden.

### ZEICHNUNGSLEGENDE

- 1: Durchflußleitung
- 2: Eingang
- 3: Durchflußraum
- 4: Ausgang
- 5: Mediumstrom
- 6: Meßgehäuse
- 7: Flansch
- 8: Dichtung
- 9: Dichtung
- 10: Dichtkragen
- 11: Rollmembrane
- 12: Kolben
- 13: Klemmstück
- 14: Abdrückstift
- 15: Kugelbüchse
- 16: Feder
- 17: Feder
- 18: Meßdorn
- 19: Meßabstand
- 20: Näherungsschalter
- 21: Büchse
- 22: Überwurfmutter
- 23: Anschluß
- 24: Wandung
- 25: Bolzen
- 26: Lufteinlaß
- 27: Ventilglocke
- 28: Ausnehmung
- 29: Unterkante
- 30: Schraube
- 31: Dichtungskragen
- 32: Rollmembrane
- 33: Kolben
- 34: Dichtung
- 35: Entlüftungsraum
- 36: Enlüftungsspalt
- 37: Lufteinlaß
- 38: Zylinderraum
- 39: Meßkammer
- 40: Ausnehmung
- 41: Pfeilrichtung
- 42: Pfeilrichtung
- 43: Position
- 44: Position
- 45: Position
- 46: Öffnungsdiagramm (Ventilglocke 27)
- 47: Öffnungsdiagramm (Kolben 12)
- 48: Position
- 49: Position
- 50: Position
- 51: Position
- 52: Position
- 53: Position
- 54: Wand
- 55: Wand
- 56: Flansch
- 57: Flansch
- 58: Zylinderkammer
- 59: Zylinder
- 60: Anschlag
- 61: Deckel

## Patentansprüche

1. Meßeinrichtung zur Erfassung der Gasbeladung oder des Dampfdruckes einer Flüssigkeit, insbesondere einer fließfähigen Kunststoffkomponete mit einer Meßkammer (39), die mit einer von der Flüssigkeit durchströmten Leitung (1,2,3,4,5) in Verbindung gebracht werden kann, die in der Meßstellung von der strömenden Flüssigkeit abgeschlossen werden kann und die einen Meßkolben (12) hat, der sich unter dem Einfluß der Entgasung des in der Meßkammer (39) eingeschlossenen Meßvolumens längs eines Meßweges verschiebt, wobei die Verschiebung des Meßkolbens (12) ein Maß für die Gasbeladung der Flüssigkeit ist, **dadurch gekennzeichnet,** daß die Meßkammer (39) in der von der Flüssigkeit durchströmten Leitung (1,2,3,4,5) liegt und eine Ventilglocke (27) aufweist, und daß die Ventilglocke (27) und/oder ein mit ihr zusammenwirkendes Teil (Flansch 7) der Meßkammer (39) gesteuert verschiebbar ist, derart, daß die Meßkammer in der Durchströmungsstellung Meßstellung ein Teil des durchströmten Raumes ist und in der davon abgeschlossen ist, in welcher Meßstellung die Flüssigkeit die abgeschlossene Meßkammer (39) umströmt.

2. Meßeinrichtung nach Anspruch 1, **dadurch gekennzeichnet,** daß sie so angeordnet ist, daß die Flüssigkeit die Meßkammer (39) im wesentlichen von unten nach oben (Pfeil 5) durchströmt.

3. Meßeinrichtung nach Anspruch 2, **dadurch gekennzeichnet,** daß am Ausgang der Meßkammer (39) gelegene Innenflächen (54,55) der Meßkammer zum Ausgang (3) hin nach oben geneigt sind.

4. Meßeinrichtung nach einem der Ansprüche 1 bis 3, **dadurch gekennzeichnet**, daß der Meßkolben (12) im Sinne einer Volumenvergrößerung der Meßkammer (39) durch die Kraft einer kalibrierten Feder (16) verschiebbar ist.

5. Meßeinrichtung nach einem der Ansprüche 1 bis 4, **dadurch gekennzeichnet**, daß die beweglichen Elemente (12,27) der Meßkammer (39) über Rollmembranen (11,32) abgedichtet sind.

## Claims

1. A measurement device to detect the gas charge or the steam pressure of fluid, in particular a flowable plastic component with a measurement chamber (39) which can be brought into connection with a conduit (1,2,3,4,5) through which the fluid flows, which in the measurement position can be closed off from the flowing fluid and which has a measurement flask (12) which moves along a measurement path under the influence of the degasification of the measurement volume enclosed in the measurement chamber (39), in which the movement of the measurement flask (12) is a measurement for the gas charge of the fluid, characterised in that the measurement chamber (39) lies in the conduit (1,2,3,4,5) through which the fluid flows and has a valve cap (27), and that the valve cap (27) and/or a part (flange 7) of the measurement chamber (39) cooperating therewith can be moved in a controlled manner, such that the measurement chamber in the through-flow position is a part of the space which is flowed through and is closed off therefrom in the measurement position, in which measurement position the fluid flows around the closed off measurement chamber (39).

2. A measurement device according to Claim 1, characterised in that it is arranged so that the fluid flows through the measurement chamber (39) substantially from the bottom to the top (arrow 5).

3. A measurement device according to Claim 2, characterised in that inner surfaces (54,55) of the measurement chamber situated at the outlet of the measurement chamber (39) are inclined upwards towards the outlet (3).

4. A measurement device according to one of Claims 1 to 3, characterised in that the measurement flask (12) is movable through the force of a calibrated spring (16) in the sense of a volume increase of the measurement chamber (39).

5. A measurement device according to one of Claims 1 to 4, characterised in that the movable members (12,27) of the measurement chamber (39) are sealed by means of roller membranes (11,32).

## Revendications

1. Dispositif de mesure pour détecter la charge gazeuse ou la pression de vapeur d'un liquide, en particulier d'un composant de matière plastique fluide, comportant une chambre de mesure (39) qui peut être reliée à une conduite (1, 2, 3, 4, 5) traversée par le liquide, qui peut être isolée par rapport au liquide qui s'écoule, en position de mesure, et qui comporte un piston de mesure (12) qui se déplace le long d'une course de mesure sous l'influence du dégazage du volume de mesure enfermé dans la chambre de mesure (39), le déplacement du piston de mesure (12) déterminant la charge gazeuse du liquide, caractérisé en ce que la chambre de mesure (39) se trouve dans la conduite (1, 2, 3, 4, 5) traversée par le liquide et comporte une cloche formant soupape (27), et en ce que la cloche formant soupape (27) et/ou un élément (bride 7) de la chambre de mesure (39) coopérant avec elle est mobile de façon commandée de telle sorte que la chambre de mesure fasse partie de l'espace traversé, en position de passage, et qu'elle en soit séparée dans la position de mesure, dans laquelle le liquide contourne la chambre de mesure (39) fermée.

2. Dispositif de mesure selon la revendication 1, caractérisé en ce qu'il est disposé de telle sorte que le liquide traverse la chambre de mesure (39) globalement de bas en haut (flèche 5).

3. Dispositif de mesure selon la revendication 2, caractérisé en ce que des surfaces intérieures (54, 55) de la chambre de mesure qui sont situées à la sortie de la chambre de mesure (39) sont inclinées vers le haut en direction de la sortie (3).

4. Dispositif de mesure selon l'une des revendications 1 à 3, caractérisé en ce que le piston de mesure (12) est mobile dans le sens d'une augmentation de volume de la chambre de mesure (39) sous l'action de la force d'un ressort calibré (16).

5. Dispositif de mesure selon l'une des revendications 1 à 4, caractérisé en ce que les éléments mobiles (12, 27) de la chambre de mesure (39) sont rendus étanches par l'intermédiaire de membranes roulées (11, 32).
